# EUROPÄISCHE PATENTSCHRIFT

(11) **EP 1 181 924 B1**
(45) Veröffentlichungstag und Bekanntmachung des Hinweises auf die Patenterteilung: **26.10.2005**
(21) Anmeldenummer: 01120056.5
(22) Anmeldetag: 21.08.2001
(51) Int. Cl.: A61K 6/083

(54) **Dentalkomposite aufweisend Hybridfüllstoffe und Verfahren zur Herstellung**
Dental composite material including a hybrid filler and method for producing the same
Composite dentaire comprenant une charge hybride et procédé de préparation

(30) Priorität: 26.08.2000 DE 10042050
(43) Veröffentlichungstag der Anmeldung: 27.02.2002
(73) Patentinhaber: DeguDent GmbH, 63457 Hanau (DE); Forschungszentrum Karlsruhe GmbH, 76133 Karlsruhe (DE)
(72) Erfinder: Alkemper, Jochen, Dr., 55270 Klein-Winternheim (DE); Binder, Joachim, 76344 Eggenstein-Leopoldshafen (DE); Rentsch, Harald, Dr., 63457 Hanau (DE); Ritzhaupt-Kleissl, Hans-Joachim, Dr., 69190 Walldorf (DE); Hausselt, Jürgen, Prof., 76726 Germersheim (DE)
(74) Vertreter: Stoffregen, Hans-Herbert

(56) Entgegenhaltungen:
- EP-A- 0 159 887
- EP-A- 0 475 239
- EP-A- 0 648 484
- WO-A-86/00021
- DE-A- 19 846 556
- US-A- 4 217 264

## Beschreibung

Die vorliegende Erfindung richtet sich auf einen Dentalwerkstoff sowie ein Verfahren zu dessen Herstellung. Ebenso betrifft die Erfindung neuartige Hybridfüllstoffe.

Insbesondere ist die Erfindung auf Dentalwerkstoffe auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 0,5-75 Gew.-% eines Hybridfüllstoffs (A) sowie bezogen auf den Dentalwerkstöff 0,0-95 Gew.-% von weiteren Füllstoffen (B) sowie 0,0-2 Gew.-% von weiteren üblichen Zuschlagstoffen gerichtet.

Auf Grund der möglichen Gesundheitsgefahren durch quecksilberhaltige Materialien (Amalgame) im Bereich der Zahnrestauration ist man verstärkt auf der Suche nach neuen quecksilberfreien Zubereitungen für diesen Anwendungszweck.

Aus der US 5426082 sind poröse Glaskeramiken bekannt, welche für die Herstellung von speziellen Katalysatoren dienen. Die dort genannten Keramiken sollen ein Mindestporenvolumen von >2000 mm³/g aufweisen. Diese hohen Porenvolumina sind für den Einsatz dieser Stoffe als Füllmaterialien in Dentalwerkstoffen nicht geeignet, da sie eine geringe Festigkeit der Füllungen bedingen.

Bei den in der EP 48 681 beschriebenen befüllten anorganischen porösen Teilchen handelt es sich um aus amorphen Gläsern bestehenden Füllstoffen. Die hier genannten Dentalwerkstoffe haben jedoch den Nachteil, daß die Partikel des benutzten Füllstoffs aufgrund Ihrer Struktur und Größe lungengängig sind und so bei Implementierung die Gefahr einer der Asbestose vergleichbaren Krankheit besteht.

Die EP-A 0 530 926 offenbart Dentalmassen aus einem polymerisierbaren Monomer und einem anorganischen Füller, der zu 20 bis 80 Gew.-% aus sphärischen anorganischen Oxidpartikeln mit einer durchschnittlichen Teilchengröße zwischen 1,0 und 5,0 µm und zu 80 - 20 Gew.-% aus sphärischen anorganischen Oxidpartikeln mit einer Teilchengröße im Bereich von mindestens 0,05 µm und weniger als 1,0 µm besteht, wobei wenigstens 5 Gew.-% der letztgenannten Komponente im Bereich von 0,05 bis 0,2 µm liegen. Bei den anorganischen Partikeln handelt es sich ausschließlich um kugelförmige Partikel anorganischer Oxide des Siliciums, Zirkoniums, Aluminiums und Titans oder Mischoxide von Elementen aus der I - IV Hauptgruppe des Periodensystems mit Silicium. Die kugelförmigen Partikel werden z. B. durch hydrolytische Polymerisation von Alkoxysilanen hergestellt und können z. B. auch mit γ-Methacryloxypropyltrimethoxysilan oberflächenbehandelt werden. Die hier genannten Füllstoffe bestehen ggf. aus Mischungen von aus einem einzigen Material hergestellten Partikeln.

In der DE 196 15 763 werden mit Monomeren beladene amorphe Siliziumdioxid-Gläser in Dentalkompositen beschrieben. Die Gläser sind homogen aufgebaut, weshalb man nicht von Hybridfüllstoffen im Sinne der Erfindung sprechen kann.

In der DE 198 46 556 werden poröse Glaskeramiken als Füllstoffkomponenten vorgeschlagen. Unter einer Glaskeramik wird dort ein teilkristalliner Stoff verstanden, welcher aus amorphen SiO₂-Kompartimenten aufgebaut ist, in welche Kompartimente aus kristallinen erfindungsgemäßen Oxiden eingebettet vorhanden sind (siehe auch Ullmann's Encyclopedia of Industrial Chemistry 5^{th} Ed., A12, S.433ff).
Mithin handelt es sich hier wiederum um ein Mischoxid also ein homogen aufgebautes Material.

Die bisher vorgeschlagenen Dentalkomposite erreichen mitunter schon die Festigkeit von Amalgamfüllungen, so daß an eine Anwendungen dieser Komposite im Kaubereich von Zähnen zu denken ist. Neben der Festigkeit ist aber auch die optisuche Qualität des Komposits zu beachten, welche sich an die natürliche Zahnumgebung möglichst unauffällig anpassen lassen soll. Darüber hinaus ist auf eine ausreichende Röntgenopazität des Füllmaterials zu achten, damit der Zahnarzt den korrekten Sitz der Füllung gut untersuchen kann.

Aufgabe der vorliegenden Erfindung war es deshalb, einen weiteren Dentalfüllstoff zu entwickeln, der im Hinblick auf die oben angesprochenen Kriterien ein optimales Ergebnis erwarten läßt.
Insbesondere sollte der Dentalwerkstoff bei vergleichbar gutem Polymerisationsschrumpf und hoher Festigkeit eine verbesserte Abrasionsfestigkeit aufweisen. Zudem sollte die durch hydrolytische Spaltung bedingte Ablösung der Polymermatrix vom anorganischen Füllstoff möglichst unterbunden werden. Der Dentalwerkstoff sollte darüber hinaus falls gewünscht röntgenopak sein sowie derart transparent, daß man in einem Arbeitsschritt den Dentalwerkstoff in die Kavität des Zahnes einbringen und mit Licht aushärten kann.

Eine weitere Aufgabe der Erfindung bestand in der Bereitstellung von bestimmten Füllstoffen, welche sich für den Einsatz in erfindungsgemäßen Dentalmaterialien eignen.

Die Aufgabe, sofern sie sich auf einen Dentalwerkstoff bezieht, wird gelöst durch einen Dentalwerkstoff mit den kennzeichnenden Merkmalen des Anspruchs 1. Die auf Anspruch 1 rückbezogenen Unteransprüche 2-8 stellen besondere Ausführungsformen des Dentalwerkstoffs unter Schutz. Ansprüche 9-11 beschreiben ein erfindungsgemäßes Verfahren zur Herstellung des Dentalwerkstoffs. Anspruch 12 ist auf einen so hergestellten Dentalwerkstoff gerichtet.

Die Aufgabe zur Angabe eines besonderen Füllmaterials wird durch Merkmale des kennzeichnenden Teils des Anspruchs 13 gelöst. Anspruch 14 ist weiterhin auf einen erfindungsgemäßen porösen Füllstoff gerichtet.

Dadurch, daß man einen Dentalwerkstoff auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 0,5-75 Gew.-% eines Hybridfüllstoffs (A) sowie bezogen auf den Dentalwerkstoff 0,0-95 Gew.-% von weiteren Füllstoffen (B) sowie 0,0-2 Gew.-% von weiteren üblichen Zuschlagstoffen (C), bereitstellt, bei dem der Hybridfüllstoff (A) aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink besteht, wobei die Füllstoffe in den Primärpartikeln als separate Teilchen statistisch verteilt vorliegen, gelangt man in nicht vorhersehbarer Weise zu überlegenen Dentalmaterialien. Insbesondere die verminderte Abrasion ist bei diesem Dentalwerkstoff hervorstechend, was dadurch bedingt ist, daß Mikrobrüche im anorganischen Teil des Füllmaterials nur kleine Ausmaße annehmen können, da sie jeweils an den Phasengrenzen der zusammengesinterten Einzelpartikel enden (Bruchlenkung). Bei vollständig aus einem Material hergestellten Füllkomponenten geht indes ein Bruch durch den gesamten Füllkörper. Eine erhöhte Abbruchtendenz von größeren Füllstoffbestandteilen und damit eine höhere Abrasion sind die Folge.

Das Verhältnis der Größen der im Dentalwerkstoff enthaltenen Partikel (Hybridfüllstoff (A) und nicht in (A) enthaltener Füllstoff (B)) ist variabel. Es sollte so eingestellt sein, daß eine möglichst dichte Packung von Füllstoffen erreicht werden kann, um einerseits den Polymerschrumpf zu minimieren und andererseits die Festigkeit des Dentalwerkstoffs zu erhöhen.
Der erfindungsgemäße Hybridfüllstoff (A) im Dentalwerkstoff besitzt eine optimale Größe von 1-200 µm, besonders bevorzugt 3-90 µm.

Das Größenverhältnis zwischen den im Hybridfüllstoff (A) enthaltenen Füllstoffen (B) und der mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink kann vom Fachmann gemäß den Anforderungen an den Hybridfüllstoff und der Praktikabilität beliebig gewählt werden. Bevorzugt ist ein Bereich der Größenverhältnisse von >1:1-1:20000, vorzugsweise 1:10-1:1000.
Auch das Massenverhältnis zwischen den im Füllstoff (A) enthaltenen Füllstoffen (B) und einem oder mehreren Primärpartikeln aus Oxiden, Fluoriden, Sulfaten, Phosphaten, Borinden, Nitriden, Carbiden und/oder Siliziden der Elemente Aluminium, Silizium, Zinn, Zirkonium, Titan und Zink kann in dem Fachmann naheliegenden Größenbereichen liegen. Bevorzugt ist ein Bereich von 25-75 Gew.-% von (B) bezogen auf das Gesamtgewicht von (A).
Das Zusammensintern der ggf. porösen Bestandteile des Hybridfüllstoffs (A) (Füllstoff (B) und Primärpartikel) kann je nach Temperatur oder Sinterdauer soweit gehen, daß der Füllstoff kompakt aufgebaut ist. Er enthält dann keine Poren mehr. Durch kürzere Sinterdauer oder Sintern bei niedrigeren Temperaturen können Porenvolumen oder Porendurchmesser jedoch vorteilhafterweise so gewählt werden, daß es speziellen Monomeren möglich ist, in die Poren einzudringen und eine interne Monomerverteilung auszubilden. Deshalb ist ein Füllstoff (A) bevorzugt, welcher ein Porenvolumen von >0-2000 mm³/g, vorzugsweise 50-1500mm³/g, aufweist. Darüber hinaus kann der Porendurchmesser des Hybridfüllstoffs (A) vorzugsweise >0-1000 nm, besonders bevorzugt 20-100 nm, betragen.
Der poröse Hybridfüllstoff (A) kann wie oben angedeutet weiterhin mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. -carbonaten beladen sein. Diese bevorzugte Ausführungsform dient dazu im Füllstoff ein Polymernetz auszubilden, welches exoseitig mit den in der Dentalmatrix vorhandenen organischen Polymeren zu reagieren im Stande ist. Man schafft so eine chemische Verzahnung der organischen Polymermatrix mit dem anorganischen Füllstoff, welche nur schwer zu zerstören ist. Eine längere Haltbarkeit der Füllungen ist die Folge. Eine vorteilhafte Vorgehensweise ist z.B. in der DE 198 46 556 aufgezeigt. Das dort Beschriebene gilt hier sinngemäß.
Ganz besonders zeichnen sich Hybridfüllstoffe (A) aus, welche Primärpartikel aus Oxiden, Fluoriden, Sulfaten und/oder Phosphaten der Elemente der I. bis V. Gruppen des Periodensystems aufweisen.

Die Erfindung richtet sich auf ein Verfahren zur Herstellung von Dentalwerkstoffen auf Basis von polymerisierbaren, beispielsweise ethylenisch ungesättigten Monomeren, Epoxiden, Ormoceren, flüssigkristallinen Monomeren, Oxethanen, Spiroorthoester bzw. - carbonaten als Bindemittel, eines Katalysators für die Kalt-, Heiß- und/oder Photopolymerisation und bezogen auf den Dentalwerkstoff 0,5-75 Gew.-%, vorzugsweise von 25 - 75 Gew-%, eines Hybridfüllstoffs (A) sowie bezogen auf den Dentalwerkstoff 0,0-95 Gew.-% von weiteren Füllstoffen (B) sowie 0-2 Gew.-% von weiteren üblichen Zuschlagstoffen (C), welches sich dadurch auszeichnet, daß der Hybridfüllstoff (A) durch Sintern einer Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink hergestellt wird.
Die Sintertemperatur kann wie oben schon angedeutet variabel gewählt werden, wobei die Sintertemperatur einen Einfluß auf das Porenvolumen und die Porencharakteristik besitzt. Die zu wählenden Temperaturen hängen von den Edukten ab. Sie liegen im allgemeinen bei >500 °C und <1500 °C, vorzugsweise bei >700 °C und <1200 °C.
Vor dem Sintern wird der Hybridfüllstoff (A) bei <200°C, vorzugsweise < 130°C, sprühgetrocknet. Geeignete Sprühtrocknungsprocedere sind dem Fachmann hinlänglich bekannt (Lukasiewicz,L.S.: J.Amer.Ceram. Soc.1998,72(4),617-624). Die Herstellung des zur Sprühtrocknung heranzuziehenden Gemisches kann dabei nach dem Fachmann bekannten Verfahren erfolgen (z.B. M. Gugleilmi et al., J. Non-Cryst. Solids 1988, 100, 292-297).
Ganz besonders geeignete Füllstoffe erhält man, wenn bei porösen Hybridfüllstoffen (A), die Poren mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. - carbonaten beladen sind. Dies kann dadurch erreicht werden, daß man die Poren mit gasförmigen oder flüssigen Monomeren belädt und die Monomere anschließend im Füllstoff ggf. polymerisiert. Man kann also optional den beladenen Hybridfüllstoff (A) in bereits auspolymerisiertem Zustand in die Dentalmasse einarbeiten oder man polymerisiert mit der Dentalmasse in der Kavität auch gleich die im Füllstoff vorhandenen Monomerbestandteile mit. Beide Vorgehensweisen sind ausführlich in der DE 198 46 556 gewürdigt.

Eine weitere Ausgestaltung der Erfindung richtet sich auf einen Dentalwerkstoff erhältlich durch ein wie eben beschriebenes erfindungsgemäßes Verfahren.

Weiterhin richtet sich die Erfindung auf die Hybridfüllstoffe (A) selbst, welche sich dadurch auszeichnen, daß diese aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink bestehen.
Bevorzugt weisen Hybridfüllstoffe (A) die weiter oben für diesen Stoff geschilderten Eigenschaften - hinsichtlich Größe, Größenverhältnis etc. - auf. Er wird bevorzugt nach einem wie weiter oben angedeuteten Sinterprozeß hergestellt. Vorzugsweise wird der Hybridfüllstoff (A) in Dentalwerkstoffen verwendet, prinzipiell kann der Füllstoff aber allgemein zur Verstärkung von Kunststoffen herangezogen werden.

In einer ganz bevorzugten Ausgestaltung der Erfindung kann der poröse Hybridfüllstoff (A) aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink bestehen und mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. -carbonaten beladen sein. Gleichfalls läßt sich dieser Füllstoff durch ein Verfahren herstellen, bei dem man den Hybridfüllstoff (A) mit gasförmigen oder flüssigen Monomeren belädt und anschließend im Füllstoff ggf. polymerisiert. Danach kann die Einarbeitung z.B. in die Dentalmasse erfolgen. Solche porösen Hybridfüllstoffe (A), welche mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. -carbonaten beladen sind, eignen sich ganz besonders vorteilhaft zur Herstellung von Dentalwerkstoffen, können jedoch ganz allgemein zur Verstärkung von Kunststoffen herangezogen werden.

### Das Bindemittel:

Als Bindemittel kommen für den Dentalwerkstoff alle diejenigen Bindemittel auf Basis eines polymerisierbaren, ethylenisch ungesättigten Monomeren in Frage, die dem Fachmann für diesen Einsatzzweck geläufig sind. Zu den mit Erfolg einsetzbaren polymerisierbaren Monomeren gehören vorzugsweise solche mit acrylischen und/öder methacrylischen Gruppen.
Insbesondere handelt es sich hierbei u. a. um Ester der α-Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z. B. N-isobutylacrylamid; Vinylester von Carbonsäuren wie z. B. Vinylacetat; Vinylether wie z. B. Butylvinylether; Mono-N-vinyl-Verbindungen wie N-Vinylpyrrolidon; und Styrol sowie seine Derivate. Besonders bevorzugt sind die nachfolgend aufgeführten mono- und polyfunktionellen (Meth)acrylsäureester und Urethan(meth)acrylsäureester.
(a) Monofunktionelle (Meth)acrylate
   Methyl(meth)acrylat, n- oder i-Propyl(meth)acrylat, n-, i- oder tert.-Butyl(meth)acrylat und 2-Hydroxyethyl (meth)acrylat.
(b) Difunktionelle (Meth)acrylate
   Verbindungen der allgemeinen Formel: 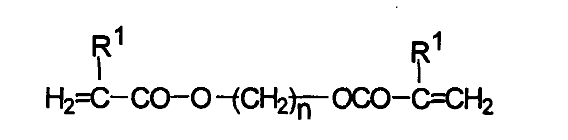 worin R¹ Wasserstoff oder Methyl ist und n eine positive ganze Zahl zwischen 3 und 20, wie z. B. Di(meth)acrylat des Propandiols, Butandiols, Hexandiols, Octandiols, Nonandiols, Decandiols und Eicosandiols,
   Verbindungen der allgemeinen Formel: 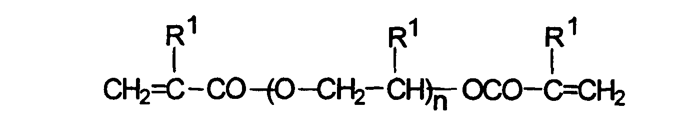 worin R¹ Wasserstoff oder Methyl ist und n eine positive ganze Zahl zwischen 1 und 14, wie z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylenglycols und Tetradecapropylenglycols; und Glycerindi(meth)acrylat, 2,2'-Bis[p-(γ-methacryloxy-β-hydroxypropoxy)-phenylpropan] oder Bis-GMA, Bisphenol-A-dimethacrylat, Neopentylglycoldi(meth)acrylat, 2,2'-Di(4-methacryloxypolyethoxyphenyl)propan mit 2 bis 10 Ethoxygruppen pro Molekül und 1,2-Bis(3-methacryloxy-2-hydroxypropoxy)butan.
(c) Tri- oder mehrfachfunktionelle (Meth)acrylate
   Trimethylolpropantri(meth)acrylat und Pentaerythritoltetra(meth)acrylat.
(d) Urethan(meth)acrylate
   Umsetzungsprodukte von 2 Mol hydroxylgruppenhaltigen (Meth)acrylatmonomer mit einem Mol Diisocyanat und Umsetzungsprodukte eines zwei NCO Endgruppen aufweisenden Urethanprepolymers mit einem methacrylischen Monomer, das eine Hydroxylgruppe aufweist, wie sie z. B. durch die allgemeine Formel wiedergegeben werden: 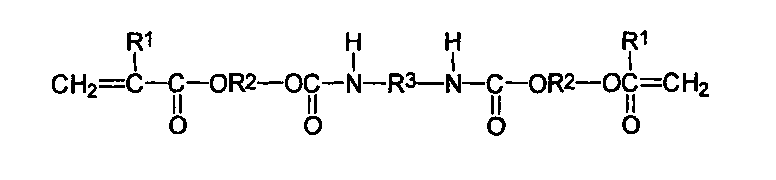 worin R¹ Wasserstoff oder eine Methylgruppe bedeutet, R² eine Alkylengruppe und R³ einen organischen Rest verkörpert.

Zu ganz besonders vorteilhaft im erfindungsgemäßen Dentalwerkstoff eingesetzten Monomeren gehören vor allem 2,2-Bis-4-(3-methacryloxy-2-hydroxypropoxy)-phenylpropan (Bis-GMA), 3,6-Dioxaoctamethylendimethacrylat (TEDMA), und/oder 7,7,9-Trimethyl-4,13-dioxo-3,14-dioxa-5,12-diaza-hexadecan-1,16-dioxy-dimethacrylat (UDMA).
Eine Beschreibung der Epoxide ist beispielsweise in DE 961 48 283 A1 gegeben.
Die Bezeichnung Ormocere beschreibt organisch modifizierte Polysiloxane wie sie beispielsweise in DE 41 33 494 C2 oder in DE 44 16 857 aufgeführt sind und für Dentalmassen verwendet werden können.
Flüssigkristalline Dentalmonomere sind in EP 0 754 675 A2 beschrieben.
Oxethane als Dentalmonomere sind in US 5 750 590 und DE 951 06 222 A1 beschrieben.
Spiroorthocarbonate werden beispielsweise in US 5 556 896 beschrieben.
Die genannten Monomeren werden entweder allein oder in Form einer Mischung von mehreren Monomeren verwendet.

### Der Katalysator:

Der Dentalwerkstoff kann je nach Art des verwendeten Katalysators heiß, kalt und/oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet.
Als Katalysatoren für die Photopolymerisation können z. B. Benzophenon und seine Derivate sowie Benzoin und seine Derivate verwendet werden. Weitere bevorzugte Photosensibilisatoren sind α-Diketone wie 9,10-Phenanthrenchinon, Diacetyl, Furil, Anisil, 4,4'-Dichlorbenzile und 4,4'-Dialkoxybenzile. Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.
Als Katalysatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z. B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht. Die Menge dieser Katalysatoren im Dentalwerkstoff liegt üblicherweise zwischen 0,01 bis 5 Gew.-%.
Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z. B. Benzoylperoxid und in die andere Paste z. B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.
Wenn man bei den letztgenannten Materialien das Amin wegläßt und als Katalysator z. B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

### Das Dentalmaterial:

Dentalwerkstoff bezeichnet im Rahmen der Erfindung Materialien für die Zahnrestauration, wie Zahnfüllung, Inlays oder Onlays, Befestigungszemente, Glasionomerzemente, Kompomere, Verblendmaterialien für Kronen und Brücken, Materialien für künstliche Zähne, in Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposite für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.
Im auspolymerisierten Zustand kann das Verhältnis der Gew.-Teile von Polymer zu Hybridfüllstoff (A) zu Füllstoffen (B) der erfindungsgemäßen Dentalfüllungen im Verhältnisbereich von 10-80 zu 20-70 zu 1-30, bevorzugt 30-50 zu 30-60 zu 5-20 liegen (in Summe 100).

### Füllstoff B:

Als (B) kommen alle dem Fachmann geläufigen Füllstoffe für Dentalwerkstoffe (z.B. zur Verbesserung der Röntgenopazität, Viskosität, Polierbarkeit, Transparenz, Festigkeit, Brechungsindex) in Frage. Zur Erzielung einer weiter erhöhten Röntgenopazität können Füllstoffe eingesetzt werden, welche z. B. in der DE-OS 35 02 594 beschrieben sind, wobei deren mittlere Primärteilchengröße 5,0 µm nicht übersteigen sollte. Gegebenenfalls können geringe Mengen an mikrofeiner, pyrogener oder naßgefällter Kieselsäure als Füllstoff (B) in den Dentalwerkstoff eingearbeitet werden.
Weiterhin kommen in Frage: Apatite gemäß der EP 0 832 636 und/oder Partikel gemäß der DE 195 08 586 und/oder DE 41 23 946, sowie Glaskeramiken nach der DE 198 46 556 und/oder. Zeolithe gemäß der DE 198 29 870 oder WO 98/49997.
Als Füllstoffe (B) werden bevorzugt Gläser, TiO₂-, Zirkonoxid- oder Fluorapatitpartikel eingesetzt.
Die Größe der Partikel liegt im allgemeinen zwischen 0,1 und 20 µm, vorzugsweise zwischen 0,5 und 5 µm. Die Form der Teilchen ist wenig ausschlaggebend. Sie kann von irregular über splittrig bis zu Plättchen-, Stäbchen und Kugelform reichen. Der Körper kann dabei amorph, teilkristallin oder vollkristallin, kompakt oder porös sein.
Er kann darüber hinaus gleichfalls aus mehreren Bestandteilen aufgebaut sein, wie beispielsweise in Kern-Schale-Bauweise. Bezüglich der Herstellung dieser Teilchen (B) sei auf die einschlägig bekannten Prozesse verwiesen, welche in der oben angegebenen Literatur angesprochen werden.

### Zuschlagstoffe C:

Unter Zuschlagstoffen sind im Rahmen der Erfindung alle dem Fachmann geläufigen Zuschlagstoffe zu verstehen, die in Dentalmassen eingearbeitet werden können, um deren Qualität zu verbessern. Als solche sind insbesondere anzusehen Färbemittel, Emulgatoren etc.

### Hybridfüllstoff A:

Wie schon berichtet wird der Hybridfüllstoff (A) durch Zusammensintern von Füllstoffen (B) und mehreren Oxiden, Fluoriden, Sulfaten, Phosphaten, Boriden, Nitriden, Carbiden und/oder Siliziden der Elemente Aluminium, Silizium, Zinn, Zirkonium, Titan und Zink hergestellt. Dabei bildet sich eine heterogene Mischung von Füllstoffen (B) in den genannten anorganischen Verbindungen (zusammengesinterte Primärpartikel) als separate Teilchen, wobei die Füllstoffe (B) in dem Teilchen statistisch verteilt vorliegen.
Ganz besonders bevorzugt ist in diesem Zusammenhang der Einsatz von Kieselsäuren jedweder Art. Eine Übersicht von möglichen Kieselsäure und ihre Herstellung ist dabei in Ullmanns, Enzyklopädie der technischen Chemie, 1982, 4. Auflage, Band 21, S. 439ff. gezeigt. Äußerst bevorzugt ist der Einsatz von Aerosil® als Sintermaterial zur Einbettung von Füllstoffen (B). Eine Übersicht über vorteilhafte Aerosil®-Arten bietet die Broschüre: Technical Bulletin Pigments, Basic Characteristics of Aerosil®, Number 11, Auflage 4, der Degussa-Hüls AG.
Die Primärpartikel sind in der Größe geringer als die der Füllstoffe (B) und besitzen vorzugsweise eine Größe von 1 bis 200 nm, besonders bevorzugt 5 bis 50 nm im Durchmesser. Es können auch unterschiedliche Primärpartikel mit Füllstoffen (B) zusammengesintert werden. Die Form der Primärpartikel ist dabei nicht ausschlaggebend - ebenso wie bei den Füllstoffen (B). Auch diese können amorph, teilkristallin oder kristallin vorliegen.

### Elemente:

Unter Elementen im Rahmen der Erfindung werden die Elemente Aluminium, Silizium, Zinn, Zirkonium, Titan und Zink verstanden, wobei jedoch Kohlenstoff, Stickstoff, Phosphor, Arsen, Antimon, Kupfer, Cadmium und Quecksilber ausgenommen sind.

### Modifikation der Füllstoffe (A) und (B):

Weitere Modifikationen der Füllstoffe (A) und/oder (B) können wie folgt aussehen:
Es können bacterizide Mittel in den porösen Hybridfüllstoff (A) implementiert werden. Als bacterizide Mittel sieht man insbesondere Hesperedin, Naringenin, Quercetin, Anisic Acid, Acetyl Coumarin, Sitosterol, Caryophyllen und Caryophyllenoxid an. Diese Verbindungen können nach den in US 4,925,660 beschriebenen Verfahren in die Poren des Hybridfüllstoffs (A) eingebaut werden.

Bevorzugt kann die innere und/oder äußere Oberfläche eines porösen Hybridfüllstoffs (A) sowie die innere und/oder äußere Oberfläche des Füllstoffs (B) vor der Einbettung in den Dentalwerkstoff optional mit dem Fachmann geläufigen Oberflächenmaterialien chemisch modifiziert werden. Dies dient u. a. zur
a) Erhöhung der mechanischen Stabilität und Hydrophobizität
   und
b) weiteren Verbesserung der Kopplung des anorganischen Füllers an die organische Matrix.

In besonderer Ausführungsform ist der Füllstoff (A und/oder B) mit Silanen der allgemeinen Formel RSi(OX)3, worin R eine Alkylgruppe mit 1 bis 18 C-Atomen und X eine Alkylgruppe mit 1 oder 2 C-Atomen ist, und/oder Metalloxiden nachbeschichtet. Zur Erhöhung der Stabilität und Hydrophobizität wird insbesondere Trimethylchlorosilan verwendet, wie beschrieben in Koyano, K. A.; Tatsumi, T.; Tanaka, Y.; Nakata, S. J. Phys. Chem. B 1997, 101, p. 9436 und Zhao, X. S.; Lu, G. Q., J. Phys. Chem. B 1998, 102, p. 1156. Wenn zur Nachbeschichtung ein Silan verwendet wird, ist es zweckmäßig, wenn dies in einer Menge von etwa 0,02 bis 2 Gewichtsprozent des Silans, berechnet als SiO₂, angewendet wird, bezogen auf das Gewicht des Füllstoffs (A) bzw. (B).
Zu besonders vorteiligen Nachbeschichtungsagenzien gehören unter anderem (CH3)3SiCl, Methyltriethoxysilan, Ethyltriethoxysilan, Octyltriethoxysilan, Octadecyltriethoxysilan, Mono- oder Polyfluoralkylethoxysilan oder auch Silane mit funktionalisierten Organogruppen, die eine spätere weitere Modifizierung durch kovalente Bindungsknüpfung in bekannter Weise ermöglichen. Im letzteren Fall sind solche Organotrialkoxysilane im Hinblick auf den erfindungsgemäßen Einsatz der Partikel als Füllstoffe in polymeren oder polymerisierbaren Systemen bevorzugt, die solche funktionelle Gruppen aufweisen, mit denen sich eine kovalente Einbindung in das Polymermaterial erreichen läßt. Beispiele hierfür sind Trimethoxyvinylsilan, H2C=C(CH3)CO2(CH2)3Si(OCH3)3, Triethoxyvinylsilan und 3-Glycidoxypropyltrimethoxysilan, sowie Silane mit Hydroxyl-, Carboxyl-, Epoxy- und Carbonsäureestergruppen tragende anorganische Reste. Die Einbindung der solcher Art modifizierten Füllstoffe in den Dentalwerkstoff geschieht dabei durch Einarbeitung der Füllstoffe in den Dentalwerkstoff und anschließende Polymerisation bei der eigentlichen Aushärtung des Dentalwerkstoffes.

An der erfindungsgemäßen Dentalmasse ist vorteilhaft, daß die Farbe, Transparenz und Röntgenopazität alleine schon durch die Zusammensetzung des Hybridfüllstoffs (A) und/oder Füllstoffs (B) eingestellt werden kann. Optional können jedoch auch weitere Metalloxide zur Nachbeschichtung eingesetzt werden, wobei dies vorzugsweise in einer Menge von 1 bis 100 Gewichtsprozent, bevorzugt 10 Gewichtsprozent, bezogen auf den Metalloxidgehalt der nicht nachbeschichteten Füllstoffe (A) bzw. (B) geschieht.
Bevorzugte Metalloxide, welche für die Nachbeschichtung eingesetzt werden, sind TiO2, Fe2O3 und/oder ZrO2. Zweckmäßig ist auch eine Ausführungsform, in der der Füllstoff (A) bzw. (B) zusätzlich mit einer Schicht aus einem polymerisierbaren organischen Bindemittel überzogen ist, welches auf mono- oder mehrfachfunktionellen (Meth)acrylaten und/oder Reaktionsprodukten aus Isocyanaten und OH-gruppenhaltigen Methacrylaten beruht.
Die Möglichkeit nach Art und Quantität im Rahmen der Erfindung die Füllstoffe (A) bzw. (B) zu variieren, ist mithin ursächlich dafür, daß man den Brechungsindex der Füllstoffe an den Brechungsindex der Polymerumgebung anpassen kann. Erst so wird gewährleistet, daß der Dentalwerkstoff als ganzes so transparent wird, daß er wie gefordert in einem Stück in der Kavität des Zahnes ausgehärtet werden kann. Eine mühsame sequentielle Auftragung und Aushärtung des Dentalmaterials kann entfallen. Bevorzugt für diesen Zweck einzusetzende Oxide sind TiO₂, ZrO₂, BaO und WO₃, ganz besonders bevorzugt wird ZrO₂ verwandt.

Ebenso wie zur Modifikation des Brechungsindex kann auch die Röntgenopazität des Füllstoffes (A) bzw. (B) in idealer Weise durch Wahl der Ausgangsmaterialien eingestellt werden. Bevorzugt für diesen Zweck einzusetzende Oxide sind TiO₂, ZrO₂, BaO, besonders bevorzugt wird ZrO₂ eingesetzt.

Mithin ist die Art und Weise der erfindungsgemäßen Füllstoffe dafür verantwortlich, daß eine überraschend hohe Verstärkung gepaart mit geringer Abrasion in den Dentalmassen erreicht wird, weshalb diese analog den bekannten Amalgamfüllungen im Kaubereich der Zähne eingesetzt werden kann.

Die nachfolgenden Beispiele sollen die Erfindung erläutern.

### Beispiele:

### 1. Aerosil/Glas-Partikel

### 1.1 Herstellung von Partikeln mit 25 Gew.-% Glas

Für die Synthese von Hybrid-Partikeln, die aus mindestens zwei verschiedenen Fraktionen bestehen, wird ein SiO₂-Sol (Teilchengröße ca. 20 nm) und eine Glas-Suspension (Teilchengröße ca. 1 µm) hergestellt. Zur Herstellung des SiO₂-Sols wird Aerosil 90® mit Hilfe eines Ultraturrax in Wasser eingearbeitet. Das Sol wird über Nacht auf einen Schüttler gestellt. Das Glas wird durch elektrostatische Stabilisierung in Wasser suspendiert. Das SiO₂-Sol und die Glas-Suspension werden im Verhältnis Aerosil 90® :Glas = 3:1 vermischt. Durch Sprühtrocknung bei Temperaturen unter 200°C und anschließender Calcinierung erhält man kugelförmige Hybrid-Partikel, bei denen die Glasteilchen annähernd homogen in der Aerosil-Matrix verteilt sind.

### 1.2 Variation des Glasanteils

Zur Herstellung von Pulver mit unterschiedlichen Glasanteilen werden SiO₂-Sole und Glas-Suspensionen in den gewünschten Verhältnissen gemischt. Nach dem Sprühtrocknen und Calcinieren bei 750°C erhält man Pulver, die sich in Abhängigkeit vom Glasanteil in der Dichte, der spezifischen Oberfläche, dem spezifischen Porenvolumen und der Porenweite unterscheiden.

| Aerosil-Anteil [Gew.-%] | Glas-Anteil [Gew.-%] | Dichte [g/cm³] | Spez. Oberfläche [m²/g] | Spez. Porenvolumen [mm³/g] | Porenweite [nm] |
|---|---|---|---|---|---|
| 90 | 10 | 2,31 | 74 | 1628 | 55 |
| 75 | 25 | 2,38 | 62 | 1510 | 57 |
| 50 | 50 | 2,46 | 45 | 1045 | 72 |
| 25 | 75 | 2,61 | 25 | 720 | 113 |
| 10 | 90 | 2,72 | 12 | 407 | 180 |
| 0 | 100 | 2,80 | 3 | 166 | 22 |

Der Einfluß des Glasanteils läßt sich folgendermaßen zusammenfassen:
- Die Dichte ändert sich linear mit dem Volumenanteil an Glas.
- Die spezifische Oberfläche und das spezifische Porenvolumen nehmen nahezu linear mit dem Massenanteil an Glas ab.
- Die Porenweite hängt stark vom Glasanteil ab.

### 1.3 Variation der Calcinierungstemperatur

Die Werkstoffeigenschaften der porösen Hybrid-Partikel lassen sich nicht nur über den Glasanteil variieren, sondern auch über die Calcinierungstemperatur bzw. -zeit. Für diese Untersuchungen wurden Hybrid-Pulver mit 10 Gew.-% und 25 Gew.-% Glas synthetisiert und anschließend sowohl bei 750°C als auch bei 1000°C calciniert.

| Aerosil-Anteil [Gew.-%] | Glas-Anteil [Gew.-%] | Calcinierungstemperatur [°C] | Dichte [g/cm³] | Spez. Oberfläche [m²/g] | Spez. Porenvolumen [mm³/g] |
|---|---|---|---|---|---|
| 90 | 10 | 750 | 2,31 | 74 | 1628 |
| 90 | 10 | 1000 | 2,32 | 61 | 1331 |
| 75 | 25 | 750 | 2,38 | 62 | 1510 |
| 75 | 25 | 1000 | 2,37 | 30 | 789 |

Die Ergebnisse dieser Untersuchungen lassen sich folgendermaßen zusammenfassen:
- Die Dichte ist nur vom Volumenanteil Glas abhängig, also nicht von der Calcinierungstemperatur.
- Die spezifische Oberfläche und das spezifische Porenvolumen nehmen sowohl mit dem Massenanteil an Glas als auch mit der Calcinierungstemperatur ab.
- Die lineare Abnahme der spez. Oberfläche bzw. des spez. Porenvolumens mit zunehmenden Glasanteil ist bei höheren Calcinierungstemperaturen steiler.

### 2. Weitere Hybrid-Partikel

### 2.1 Hybrid-Partikel mit Mischoxid-Aerosilen

Anstelle der Aerosile können auch Mischoxid-Aerosile verwendet werden, die z.B. Al₂O₃ enthalten. Hierfür wurden zwei SiO₂/Al₂O₃-Sole unterschiedlicher Zusammensetzung hergestellt. Als Edukte dienten MOX F223 (16 Gew.-% Al₂O₃) und VP MOX 90 (61 Gew.-% Al₂O₃). Die MOX-Aerosile werden mit Hilfe eines Ultraturrax in Wasser eingearbeitet und die Sole über Nacht auf einen Schüttler gestellt. Die SiO₂/Al₂O₃-Sole werden jeweils mit einer Glas-Suspension im Verhältnis von MOX Aerosil:Glas = 1:1 vermischt. Durch Sprühtrocknung bei Temperaturen unter 200°C und anschließender Calcinierung erhält man Hybrid-Partikel, bei denen die Glasteilchen annähernd homogen in der MOX Aerosil-Matrix verteilt sind.

### 2.2 Hybrid-Partikel mit Glas, Fluorapatit und Zirkoniumdioxid

Anstelle eines amorphen Glases mit Teilchengrößen von ca. 1 µm können auch feinere Gläser verwendet werden, aber auch kristalline Verbindungen wie Fluorapatit oder Zirkoniumdioxid. Hierfür wurden Aerosil 90® -Sole mit einer Glas-Suspension (Teilchengröße ca. 0,7 µm) im Verhältnis von Aerosil 90® :Glas = 1:1 und mit Suspenisonen von Fluorapatit bzw. Zirkoniumdioxid im Verhältnis von Aerosil 90® :kristalline Verbindung = 3:1 vermischt. Durch Sprühtrocknung bei Temperaturen unter 200°C und anschließender Calcinierung erhält man kugelförmige Hybrid-Partikel, bei denen das Glas, der Fluorapatit oder das Zirkoniumdioxid annähernd homogen in der Aerosil-Matrix verteilt sind.

## Patentansprüche

1. Dentalwerkstoff auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 0,5-75 Gew.-% eines Hybridfüllstoffs (A) sowie bezogen auf den Dentalwerkstoff 0,0-95 Gew.-% von weiteren Füllstoffen (B) sowie 0,0-2 Gew.-% von weiteren üblichen Zuschlagstoffen (C),
**dadurch gekennzeichnet,**
**dass** der Hybridfüllstoff (A) aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink besteht, wobei die Füllstoffe in den Primärpartikeln als separate Teilchen statistisch verteilt vorliegen.

2. Dentalwerkstoff nach Anspruch 1,
**dadurch gekennzeichnet, daß**
der Hybridfüllstoff (A) eine Größe von 1-200 µm, vorzugsweise 3-90 µm, hat.

3. Dentalwerkstoff nach Anspruch 1 und/oder 2,
**dadurch gekennzeichnet, daß**
das Größenverhältnis zwischen den im Füllstoff (A) enthaltenen Füllstoffen (B) und den Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink im Bereich von >1:1 - 1:20000, vorzugsweise 1:10 - 1:1000, liegt.

4. Dentalwerkstoff nach Anspruch 1, 2 und/oder 3,
**dadurch gekennzeichnet, daß**
das Massenverhältnis zwischen den im Füllstoff (A) enthaltenden Füllstoffen (B) und den Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink im Bereich von 25-75 Gew.-% von (B) bezogen auf das Gesamtgewicht von (A) liegt.

5. Dentalwerkstoff nach einem oder mehreren der Ansprüche 1 bis 4,
**dadurch gekennzeichnet, daß**
das Porenvolumen des Hybridfüllstoffs (A) >0-2000 mm³/g, vorzugsweise 50-1500 mm³/g, beträgt.

6. Dentalwerkstoff nach einem oder mehreren der Ansprüche 1 bis 5,
**dadurch gekennzeichnet, daß**
der Porendurchmesser des Hybridfüllstoffs (A) >0-1000 nm, vorzugsweise 20-100 nm, beträgt.

7. Dentalwerkstoff nach einem oder mehreren der Ansprüche 5 bis 6,
**dadurch gekennzeichnet, daß**
der Hybridfüllstoff (A) mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. -carbonaten beladen ist.

8. Dentalwerkstoff nach einem oder mehreren der Ansprüche 1 bis 7,
**dadurch gekennzeichnet, daß**
der Hybridfüllstoff (A) Primärpartikeln aus Oxiden, Fluoriden, Sulfaten und/oder Phosphaten der Elemente Aluminium, Silizium, Zinn, Zirkonium, Titan und Zink aufweist.

9. Verfahren zur Herstellung von Dentalwerkstoffen auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 0,5-75 Gew.-% eines Hybridfüllstoffs (A) sowie bezogen auf den Dentalwerkstoff 0,0-95 Gew.-% von weiteren Füllstoffen (B) sowie 0,0-2 Gew.-% von weiteren üblichen Zuschlagstoffen (C),
**dadurch gekennzeichnet,**
**dass** man den Hybridfüllstoff (A) bei <130° C sprühtrocknet und anschließend durch Sintern einer Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink herstellt.

10. Verfahren nach Anspruch 9,
**dadurch gekennzeichnet, daß**
man den Hybridfüllstoff (A) bei >500°C und <1500°C, vorzugsweise bei >700°C und <1200°C, sintert.

11. Verfahren nach einem oder mehreren der Ansprüche 9 bis 10 zu Herstellung von porösen Hybridfüllstoffen (A), welche mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw. -carbonaten beladen sind,
**dadurch gekennzeichnet, daß**
man diese mit gasförmigen oder flüssigen Monomeren belädt und anschließend im Füllstoff ggf. polymerisiert.

12. Dentalwerkstoff erhältlich durch ein Verfahren nach Anspruch 9.

13. Hybridfüllstoff (A),
**dadurch gekennzeichnet, dass**
dieser aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink besteht, wobei die Füllstoffe in den Primärpartikeln als separate Teilchen statistisch verteilt vorliegen.

14. Poröser Hybridfüllstoff (A), bestehend aus einer gesinterten heterogenen Mischung aus Füllstoffen (B) und mehreren Primärpartikeln aus Oxiden, Fluoriden, Sulfaten, Phosphaten, Boriden, Nitriden, Carbiden und/oder Siliziden der Elemente Aluminium, Silizium, Zinn, Zirkonium, Titan und Zink, wobei die Füllstoffe in den Primärpartikeln als separate Teilchen statistisch verteilt vorliegen,
**dadurch gekennzeichnet, dass**
dieser mit polymerisierbaren oder polymerisierten, beispielsweise ethylenisch ungesättigten Monomeren, Epoxiden, Ormoceren, flüssigkristallinen Monomeren, Oxethanen, Spiroorthoester bzw.-carbonaten beladen ist.

## Claims

1. Dental material based on polymerisable, for example ethylenically unsaturated, monomers, epoxides, ormocers, liquid crystalline monomers, oxethanes, spiroorthoesters or -carbonates as binder, a catalyst for cold-, heat- and/or photo-polymerisation and 0.5-75 wt.% in relation to the dental material of a hybrid filler (A) and 0.0-95 wt.% in relation to the dental material of other fillers (B) together with 0.0-2 wt.% of other conventional additives (C),
**characterised in that**
hybrid filler (A) consists of a sintered heterogeneous mixture of fillers (B) and a plurality of primary particles of oxides, fluorides, sulfates, phosphates, borides, nitrides, carbides and/or silicides of the elements aluminium, silicon, tin, zirconium, titanium and zinc, the fillers being randomly distributed in the primary particles as separate particles.

2. Dental material according to claim 1,
**characterised in that**
the size of hybrid filler (A) is 1-200 µm, preferably 3-90 µm.

3. Dental material according to claim 1 and/or 2,
**characterised in that**
the size ratio between the fillers (B) contained in filler (A) and the primary particles of oxides, fluorides, sulfates, phosphates, borides, nitrides, carbides and/or silicides of the elements aluminium, silicon, tin, zirconium, titanium and zinc is in the range >1:1 - 1:20000, preferably 1:10 - 1:1000.

4. Dental material according to claim 1, 2 and/or 3,
**characterised in that**
the ratio of the mass of the fillers (B) contained in filler (A) to the primary particles of oxides, fluorides, sulfates, phosphates, borides, nitrides, carbides and/or silicides of the elements aluminium, silicon, tin, zirconium, titanium and zinc is in the range 25-75 wt.% of (B) in relation to the total weight of (A).

5. Dental material according to one or more of claims 1 to 4,
**characterised in that**
the pore volume of hybrid filler (A) is >0-2000 mm³/g, preferably 50-1500 mm³/g.

6. Dental material according to one or more of claims 1 to 5,
**characterised in that**
the pore diameter of hybrid filler (A) is >0-1000 nm, preferably 20-100 nm.

7. Dental material according to one or more of claims 5 to 6,
**characterised in that**
hybrid filler (A) is loaded with polymerisable or polymerised, for example ethylenically unsaturated monomers, epoxides, ormocers, liquid crystalline monomers, oxethanes, spiroorthoesters or -carbonates.

8. Dental material according to one or more of claims 1 to 7,
**characterised in that**
hybrid filler (A) contains primary particles of oxides, fluorides, sulfates and/or phosphates of the elements aluminium, silicon, tin, zirconium, titanium and zinc.

9. Process for the production of dental materials based on polymerisable, for example ethylenically unsaturated monomers, epoxides, ormocers, liquid crystalline monomers, oxethanes, spiroorthoesters or
-carbonates as binder, a catalyst for cold-, heat-, or photo-polymerisation and 0.5-75 wt.% in relation to the dental material of a hybrid filler (A) and
0.0-95 wt.% in relation to the dental material of other fillers (B) and 0.0-2 wt.% of other conventional additives (C),
**characterised in that**
hybrid filler (A) is spray dried at <130°C and then produced by sintering a mixture of fillers (B) and one or more primary particles of oxides, fluorides, sulfates, phosphates, borides, nitrides, carbides and/or silicides of the elements aluminium, silicon, tin, zirconium, titanium and zinc.

10. Process according to claim 9,
**characterised in that**
hybrid filler (A) is sintered at >500°C and <1500°C, preferably at >700°C and <1200°C.

11. Process according to one or more of claims 9 to 10 for the production of porous hybrid fillers (A) which are loaded with polymerisable or polymerised, for example ethylenically unsaturated, monomers, epoxides, ormocers, liquid crystalline monomers, oxethanes, spiroorthoesters or -carbonates,
**characterised in that**
these are loaded with gaseous or liquid monomers and then optionally polymerised in the filler.

12. Dental material which is obtained by a process according to claim 9.

13. Hybrid filler (A),
**characterised in that**
it consists of a sintered heterogeneous mixture of fillers (B) and a plurality of primary particles of oxides, fluorides, sulfates, phosphates, borides, nitrides, carbides and/or silicides of the elements aluminium, silicon, tin, zirconium, titanium and zinc, the fillers being randomly distributed in the primary particles as separate particles.

14. Porous hybrid filler (A) consisting of a sintered heterogeneous mixture of fillers (B) and a plurality of primary particles of oxides, fluorides, sulfates, phosphates, borides, nitrides, carbides and/or silicides of the elements aluminium, silicon, tin, zirconium, titanium and zinc, the fillers being randomly distributed in the primary particles as separate particles,
**characterised in that**
it is loaded with polymerisable or polymerised, for example ethylenically unsaturated, monomers, epoxides, ormocers, liquid crystalline monomers, oxethanes, spiroorthoesters or -carbonates.

## Revendications

1. Matériau dentaire à base de monomères polymérisables, par exemple éthyléniquement insaturés, d'époxydes, d'ormocers, de monomères à cristaux liquides, d'oxéthanes, de spiroortho-esters, ou -carbonates comme liants, d'un catalyseur pour la polymérisation à froid, à chaud et/ou la photopolymérisation et, par rapport au matériau dentaire, de 0,5 à 75 % en poids d'une charge hybride (A) ainsi que, par rapport au matériau dentaire, de 0,0 à 95 % en poids d'autres charges (B) ainsi que de 0,0 à 2 % en poids d'autres additifs habituels (C),
**caractérisé en ce que**
la charge hybride (A) est constituée d'un mélange hétérogène fritté de charges (B) et de plusieurs particules primaires d'oxydes, de fluorures, de sulfates, de phosphates, de borures, de nitrures, de carbures et/ou de siliciures des éléments aluminium, silicium, étain, zirconium, titane et zinc, les charges se trouvant réparties de manière statistique dans les particules primaires sous forme de petits morceaux séparés.

2. Matériau dentaire selon la revendication 1,
**caractérisé en ce que**
la charge hybride (A) a une taille de 1 à 200 µm, de préférence de 3 à 90 µm.

3. Matériau dentaire selon la revendication 1 et/ou 2,
**caractérisé en ce que**
le rapport de taille entre les charges (B) contenues dans la charge (A) et les particules primaires d'oxydes, de fluorures, de sulfates, de phosphates, de borures, de nitrures, de carbures et/ou de siliciures des éléments aluminium, silicium, étain, zirconium, titane et zinc se situe dans un intervalle allant de > 1 : 1 à 1 : 20000, de préférence de 1 : 10 à 1 : 1000.

4. Matériau dentaire selon les revendications 1, 2 et/ou 3,
**caractérisé en ce que**
le rapport de masses entre les charges (B) contenues dans la charge (A) et les particules primaires d'oxydes, de fluorures, de sulfates, de phosphates, de borures, de nitrures, de carbures et/ou de siliciures des éléments aluminium, silicium, étain, zirconium, titane et zinc se situe dans un intervalle de 25 à 75 % en poids de (B) par rapport au poids total de (A).

5. Matériau dentaire selon une ou plusieurs des revendications 1 à 4,
**caractérisé en ce que**
le volume des pores de la charge hybride (A) est de >0-2000 mm³/g, de préférence de 50-1500 mm³/g.

6. Matériau dentaire selon une ou plusieurs des revendications 1 à 5,
**caractérisé en ce que**
le diamètre des pores de la charge hybride (A) est de >0-1000 nm, de préférence de 20-100 nm.

7. Matériau dentaire selon une ou plusieurs des revendications 5 à 6,
**caractérisé en ce que**
la charge hybride (A) est chargée avec des monomères polymérisables ou polymérisés, par exemple éthyléniquement insaturés, des époxydes, des ormocers, des monomères à cristaux liquides, des oxéthanes, des spiroorthoesters, ou respectivement -carbonates.

8. Matériau dentaire selon une ou plusieurs des revendications 1 à 7,
**caractérisé en ce que**
la charge hybride (A) présente des particules primaires d'oxydes, de fluorures, de sulfates et/ou de phosphates des éléments aluminium, silicium, étain, zirconium, titane et zinc.

9. Procédé de fabrication de matériaux dentaires à base de monomères polymérisables, par exemple éthyléniquement insaturés, d'époxydes, d'ormocers, de monomères à cristaux liquides, d'oxéthanes, de spiroorthoesters, ou respectivement -carbonates comme liants, d'un catalyseur pour la polymérisation à froid, à chaud et/ou la photopolymérisation et, par rapport au matériau dentaire, de 0,5 à 75 % en poids d'une charge hybride (A) ainsi que, par rapport au matériau dentaire, de 0,0 à 95 % en poids d'autres charges (B) ainsi que de 0,0 à 2 % en poids d'autres additifs habituels (C),
**caractérisé en ce qu'**
on atomise la charge hybride (A) à moins de 130° C, puis
on procède à la fabrication par frittage d'un mélange de charges (B) et de plusieurs particules primaires d'oxydes, de fluorures, de sulfates, de phosphates, de borures, de nitrures, de carbures et/ou de siliciures des éléments aluminium, silicium, étain, zirconium, titane et zinc.

10. Procédé selon la revendication 9,
**caractérisé en ce qu'**
on fritte la charge hybride (A) à plus de 500 °C et à moins de 1500 °C, de préférence à plus de 700 °C et à moins de 1200 °C.

11. Procédé selon une ou plusieurs des revendications 9 à 10 de fabrication de charges hybrides poreuses (A), qui sont chargées avec des monomères polymérisables ou polymérisés, par exemple éthyléniquement insaturés, des époxydes, des ormocers, des monomères à cristaux liquides, des oxéthanes, des spirorothoesters, ou respectivement -carbonates,
**caractérisé en ce qu'**
on les charge avec des monomères gazeux ou liquides, puis le cas échéant on les polymérise dans la charge.

12. Matériau dentaire que l'on peut obtenir par un procédé selon la revendication 9.

13. Charge hybride (A),
**caractérisée en ce qu'**
elle se compose d'un mélange hétérogène fritté de charges (B) et de plusieurs particules primaires d'oxydes, de fluorures, de sulfates, de phosphates, de borures, de nitrures, de carbures et/ou de siliciures des éléments aluminium, silicium, étain, zirconium, titane et zinc, les charges se présentant sous forme statistiquement répartie en petits morceaux séparés dans les particules primaires.

14. Charge hybride poreuse (A) constituée d'un mélange hétérogène fritté de charges (B) et de plusieurs particules primaires d'oxydes, de fluorures, de sulfates, de phosphates, de borures, de nitrures, de carbures et/ou de siliciures des éléments aluminium, silicium, étain, zirconium, titane et zinc, les charges se présentant sous forme répartie en petits morceaux séparés dans les particules primaires,
**caractérisée en ce qu'**
elle est chargée avec des monomères polymérisables ou polymérisés, par exemple éthyléniquement insaturés, des époxydes, des ormocers, des monomères à cristaux liquides, des oxéthanes, des spiroorthoesters, ou respectivement -carbonates.
